# EUROPEAN PATENT APPLICATION

(11) **EP 2 915 493 A1**
(43) Date of publication of application: **09.09.2015**
(21) Application number: 15158112.1
(22) Date of filing: 06.03.2015
(51) Int. Cl.: A61B 17/16

(54) **CONNECTION DEVICE FOR A MILLING TOOL FOR PROSTHETIC SURGERY**

(30) Priority: 06.03.2014 IT UD20140042
(71) Applicant: HPF S.P.A., 33034 Fagagna (UD) (IT)
(72) Inventor: Tommaso, Lualdi, 33034 Fagagna (UD) (IT)
(74) Representative: Petraz, Gilberto Luigi

(57) **Abstract**

Connection device to releasably connect a milling tool (11) for prosthetic surgery and a manipulator device. The connection device comprises a positioning unit (13) configured to receive an attachment unit (11d) of the milling tool (11) and to be connected to the manipulator device to be made to rotate around a milling axis (X). The connection device also comprises an anchoring unit (17), configured to anchor axially and angularly releasably the milling tool (11) to the positioning unit (13). The anchoring unit (17) is configured mobile with respect to the positioning unit (13), axially along said milling axis (X) and angularly around it, to selectively define a condition of complete releasable clamping of the attachment unit (11d) to the positioning unit (13) and a condition of complete detachment.

## Description

### FIELD OF THE INVENTION

Forms of embodiment described here concern a connection device for connecting a milling tool for prosthetic surgery operations and a corresponding manipulator device. The milling tool in particular can be used for making a bone seating, for example for installing an acetabular prosthesis of the hip, or a shoulder prosthesis or other.

### BACKGROUND OF THE INVENTION

Milling tools in general are known, used during prosthetic surgery operations and configured to achieve coordinated and mating bone seatings, suitable for the disposition and implant of corresponding surgical prosthesis components, for example for installation of acetabular cups of hip prostheses.

Known milling tools provide a milling cap mating with the bone seating to be made, and on which a plurality of through apertures are made, provided with cutting edges, to perform a mechanical action of excavation on the bone.

In this way, by rotating around a milling axis, it is possible to perform a mechanical action of removing the bone material, and an impression is created with the desired sizes and conformation, defining a bone seating that substantially corresponds to the milling cap.

It is known that such milling tools are connected to manipulator devices which can be both manual and automatic, by means of a connection device able to cooperate with an attachment unit provided on the milling tool. The connection devices and attachment units obtain a mechanical connection of the parts, in which a male element, normally provided on the attachment unit, cooperates through shaped coupling with a female element, also called connection seating, normally provided on the connection device.

The mechanical connection can provide, for example, to use systems with a straight or bayonet joint. In particular, although it can generally guarantee a better transmission of the rotation compared with the straight joint, the bayonet joint can have the disadvantage that, at the end of rotation, or in the event of a sudden drop in the rotation speed, the inertia at play can cause a counter-rotation of the tool with respect to the manipulator device and therefore cause the milling tool to accidentally become detached from the manipulator device.

Another disadvantage of known forms of embodiment is that the connection device is typically a single body, or in any case provided with parts that cannot be completely removed, and this can complicate the steps of cleaning the connection device after it has been surgically used, and make the cleaning laborious and ineffectual.

Documents US-A-2007/191854, WO-A-2010/004267, US-B-8,475,460 and WO-A-03/094739 describe known connection devices for connecting a drive member to a tool or instrument for orthopedic and prosthetic surgery, for example a surgical miller or reamer.

There is therefore a need to perfect a connection device for connecting a manipulator device to a milling tool for prosthetic surgery that can overcome at least one of the disadvantages of the state of the art.

In particular, one purpose of the present invention is to obtain a connection device that allows to obtain a stable and secure connection of the milling tool and the manipulator device, with a solution that is at the same time simple to achieve and intuitive to use.

Another purpose of the present invention is to allow the cleaning steps to be carried out completely on all the parts that make up the connection device, without forming dead zones that are not adequately cleaned.

The Applicant has devised, tested and embodied the present invention to overcome the shortcomings of the state of the art and to obtain these and other purposes and advantages.

### SUMMARY OF THE INVENTION

The present invention is set forth and characterized in the independent claim, while the dependent claims describe other characteristics of the invention or variants to the main inventive idea.

In accordance with the above purposes, forms of embodiment described here concern a connection device for releasably connecting a milling tool for prosthetic surgery and a manipulator device which overcomes the limits of the state of the art and eliminates the defects present therein. In some forms of embodiment, the connection device comprises a positioning unit configured to receive and stably position an attachment unit of the milling tool and to be connected to the manipulator device so as to be made to rotate around a milling axis, and an anchoring unit configured to anchor the milling tool to the positioning unit axially, angularly and releasably.

Thanks to the anchoring unit, a stable and secure connection of the milling tool and manipulator device is obtained, and at the same time a solution that is simple to make and intuitive to use is achieved.

Furthermore, by providing an anchoring unit and a positioning unit of the milling tool, it is possible to completely dismantle the connection device, allowing to complete the cleaning steps on all the parts of which it consists, without forming dead zones that are not adequately cleaned.

According to some forms of embodiment, the anchoring unit comprises a mobile anchoring body provided with at least one axial holding seating configured to axially receive and clamp the attachment unit of the milling tool.

According to some forms of embodiment, the mobile anchoring body is also provided with at least one angular holding seating configured to cooperate with the positioning unit in an operative angular and axial clamping position in which it defines a complete releasable clamping condition of the attachment unit to the positioning unit, so as to prevent any unwanted rotation of the mobile anchoring body.

The present invention also concerns a surgical instrument for prosthetic surgery comprising a milling tool for prosthetic surgery, a manipulator device configured to make the milling tool rotate around a milling axis and a connection device according to the forms of embodiment described here, to releasably connect the milling tool and the manipulator device.

These and other aspects, characteristics and advantages of the present disclosure will be better understood with reference to the following description, drawings and attached claims. The drawings, which are integrated and form part of the present description, show some forms of embodiment of the present invention, and together with the description, are intended to describe the principles of the disclosure.

The various aspects and characteristics described in the present description can be applied individually where possible. These individual aspects, for example aspects and characteristics described in the attached dependent claims, can be the object of divisional applications.

It is understood that any aspect or characteristic that is discovered, during the patenting process, to be already known, shall not be claimed and shall be the object of a disclaimer.

### BRIEF DESCRIPTION OF THE DRAWINGS

These and other characteristics of the present invention will become apparent from the following description of some forms of embodiment, given as a non-restrictive example with reference to the attached drawings wherein:
- fig. 1 is a perspective view of some forms of embodiment of a connection device according to the present description for connecting a milling tool and a manipulator device;
- fig. 2 is an exploded perspective view of forms of embodiment of a connection device according to the present description;
- fig. 3 is a first lateral view of a connection device according to the present description;
- fig. 4 is a second lateral view of a connection device according to the present description;
- fig. 5 is a section of a part of a connection device according to the present description;
- fig. 6 is a perspective view of a connection device according to other forms of embodiment, for connecting a milling tool and a manipulator device.

To facilitate comprehension, the same reference numbers have been used, where possible, to identify identical common elements in the drawings. It is understood that elements and characteristics of one form of embodiment can conveniently be incorporated into other forms of embodiment without further clarifications.

### DETAILED DESCRIPTION OF SOME FORMS OF EMBODIMENT

We shall now refer in detail to the various forms of embodiment of the present invention, of which one or more examples are shown in the attached drawing. Each example is supplied by way of illustration of the invention and shall not be understood as a limitation thereof. For example, the characteristics shown or described insomuch as they are part of one form of embodiment can be adopted on, or in association with, other forms of embodiment to produce another form of embodiment. It is understood that the present invention shall include all such modifications and variants.

Before describing these forms of embodiment, we must also clarify that the present description is not limited in its application to the details of construction and disposition of the components as described in the following description using the attached drawings. The present description can include other forms of embodiment and can be achieved or put into practice in other, varied ways. Furthermore, we must clarify that the phraseology and terminology used here is for the purposes of description, and shall not be considered as limiting.

Unless otherwise specified, terms such as "mounted", "connected", "supported" and "coupled" and their variations are used in the widest sense, and include both direct and indirect assemblies, connections, supports and couplings.

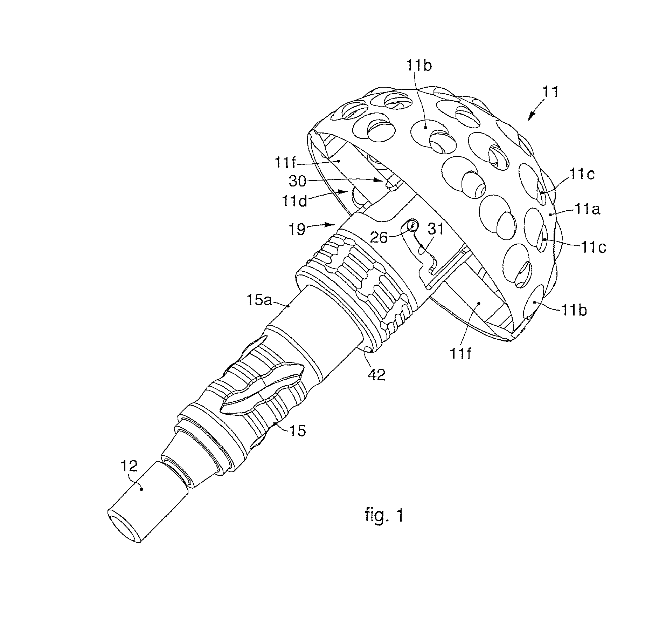

Figs. 1-6 are used to describe forms of embodiment of a connection device 10 according to the present description, configured to obtain a releasable connection of a milling tool 11 for prosthetic surgery and a manipulator device, not shown in the drawings for convenience, by means of which the milling tool 11 is made to rotate around a milling axis X.

According to the present description, the connection device 10 includes a positioning unit 13 to receive an attachment unit 11d of the milling tool 11 and to be connected to the manipulator device to be made to rotate around the milling axis X.

Furthermore, the connection device 10 includes a mechanical anchoring unit 17, to anchor the milling tool 11 to the positioning unit 13 axially, angularly and releasably.

In particular, the anchoring unit 17 is configured mobile with respect to the positioning unit 13, axially along the milling axis X and angularly around it, to selectively define a condition of complete releasable clamping of the attachment unit 11 d to the positioning unit 13 and a condition of complete detachment.

In possible implementations, the anchoring unit 17 is mobile axially and angularly along the positioning unit 13 between a distal non-operative position, in which it can be completely detached and separated, that is, put in a condition of complete dis-assembly, and a proximal insertion condition, in which it can axially receive the attachment unit 11d of the milling tool 11.

The proximal insertion position can also allow a rapid substitution of the milling tool 11 in the operating theater, without having to assume each time the distal non-operative position, since the radial arms of the cap 11f are in any case free to slide axially along the positioning unit 13 and to disconnect from it.

Furthermore, in possible implementations, the anchoring unit 17 is further mobile angularly around the positioning unit 13, between the proximal insertion position and an operative angular and axial clamping position, in which said condition of complete releasable clamping of the attachment unit 11d to the positioning unit 13 is defined. In this operative angular and axial clamping position, the anchoring unit 17 blocks any axial dis-insertion movement of the attachment unit 11d from the positioning unit 13 and is also rotationally clamped with respect to the positioning unit 13.

The axial and angular holding selectively obtainable by means of the anchoring unit 17 guarantees stability and safety of the connection of the milling tool 11 to the manipulator device, in particular preventing phenomena of counter-rotation and axial dis-insertion. Furthermore, thanks to the fact that the anchoring unit 17 is completely detachable and removable from the positioning unit 13, it is possible to completely dis-assemble the connection device 10 in question, to obtain a complete and uniform cleaning of all the parts of which it consists, without any dead zones.

According to some forms of embodiment, which can be combined with all the forms of embodiment described here, the anchoring unit 17 can include a mobile anchoring body 19 provided with at least one axial holding seating 30 configured to receive and axially clamp the attachment unit 11d, in particular a corresponding radial arm of the cap 11 f, of the milling tool 11 and thus prevent any unwanted axial dis-insertion in the operative angular and axial clamping position.

Furthermore, according to other forms of embodiment, which can be combined with all the forms of embodiment described here, the mobile anchoring body 19 can be provided with at least one angular holding seating 31 configured to cooperate with the positioning unit 13 in the operative angular and axial clamping position, so as to prevent any unwanted rotation of the mobile anchoring body 19 and thus prevent the latter from returning to the proximal insertion position, in which it would be free to detach itself axially from the attachment unit 11d.

According to some forms of embodiment, which can be combined with all the forms of embodiment described here, the anchoring unit 17 can include an elastic element 18 configured to act elastically along the milling axis X and to abut on one side against the mobile anchoring body 19 and on the other side against the positioning unit 13. Examples of elastic element 18 can be a spring, in particular a helical spring, for example made of harmonic steel for springs, or an element made of elastic material, such as an elastic polymer of synthetic or natural origin, possibly with a shape that is susceptible to non-permanent elastic deformation following stress, for example accordion shaped.

In possible implementations, the elastic element 18 can be configured to thrust normally against the mobile anchoring body 19 in the direction of the milling axis X, so as to tend to distance it from the attachment unit 11d positioned by the positioning unit 13.

Consequently, thanks to the elastic element 18, the axial holding seating 30 and the angular holding seating 31 of the mobile anchoring body 19 are kept constantly thrust elastically against the attachment unit 11d and the positioning unit 13.

When, for example, an operator applies on the mobile anchoring body 19 an axial thrust contrary to the elastic thrust of the elastic element 18, that is, it thrusts the mobile anchoring body 19 toward the attachment unit 11d, it is possible to release the attachment unit 11d and the positioning unit 13 from the axial holding seating 30 and the angular holding seating 31.

It is therefore possible to rotate the mobile anchoring body 19 from the operative angular and axial clamping position to the proximal insertion position, thus freeing the attachment unit 11 d so that the milling tool 11 can be separated and removed axially. Subsequently, it is possible to perform another rotation and make the mobile anchoring body 19 slide axially from the proximal insertion position to the distal non-operative position, possibly removing it completely from the positioning unit 13, to obtain a complete dis-assembly of the connection device 10.

In this way, the elastic element 18 can cooperate with the mobile anchoring body 19 and the positioning unit 13 to allow both the elastic axial clamping of the operative angular and axial clamping position and also the passage between the proximal insertion position and the distal non-operative position by means of an automatic elastic movement.

According to some forms of embodiment, which can be combined with all the forms of embodiment described here, the positioning unit 13 includes a positioning block 14 configured to receive the attachment unit 11d of the milling tool 11.

In particular, in possible implementations, the positioning block 14 is configured to draw the attachment unit 11 d into rotation and prevent any relative rotation between the milling tool 11 and the positioning unit 13 around the milling axis X. In other words, when the positioning unit 13 is made to rotate by the manipulator device, the positioning block 14 and the milling tool 11 rotate solidly with each other and, at both beginning and end of the rotation, there is no relative rotation between the two.

According to some forms of embodiment, which can be combined with all the forms of embodiment described here, the positioning unit 13 can also include a connection element 12, such as a tang, a cylinder or similar connection element, for connection to the manipulator device and to transmit the mechanical force imparted by the manipulator device to the milling tool 11 to allow it to be used.

In possible implementations, the anchoring unit 17 and the positioning unit 13 are reciprocally configured to allow both sliding of the anchoring unit 17 with respect to the positioning unit 13 along the milling axis X, and also a rotation of the anchoring unit 17 with respect to the positioning unit 13 around the milling axis X.

To this purpose, for example, the positioning unit 13 can include an intermediate sliding guide body 16 to cooperate with the anchoring unit 17.

In possible implementations, in the proximal insertion position, the anchoring unit 17, and in particular the mobile anchoring body 19, can be positioned above the positioning block 14, in particular it can be coaxial with it, surrounding it externally, to effectively hold the milling tool 11 both angularly and axially. According to some forms of embodiment, the milling tool 11 can be for example an acetabular miller for prosthetic surgery, including a milling cap 11 a provided with one or more cutting edges 11b associated with holes or apertures 11 c, for example to perform the operations to cut and remove the bone material.

In possible forms of embodiment, the attachment unit 11 d can include for example two radial arms of the cap 11f which are aligned, that is, they form an angle of 180° with respect to each other and extend from the center toward the periphery, joining with the milling cap 11a (see fig. 1 for example).

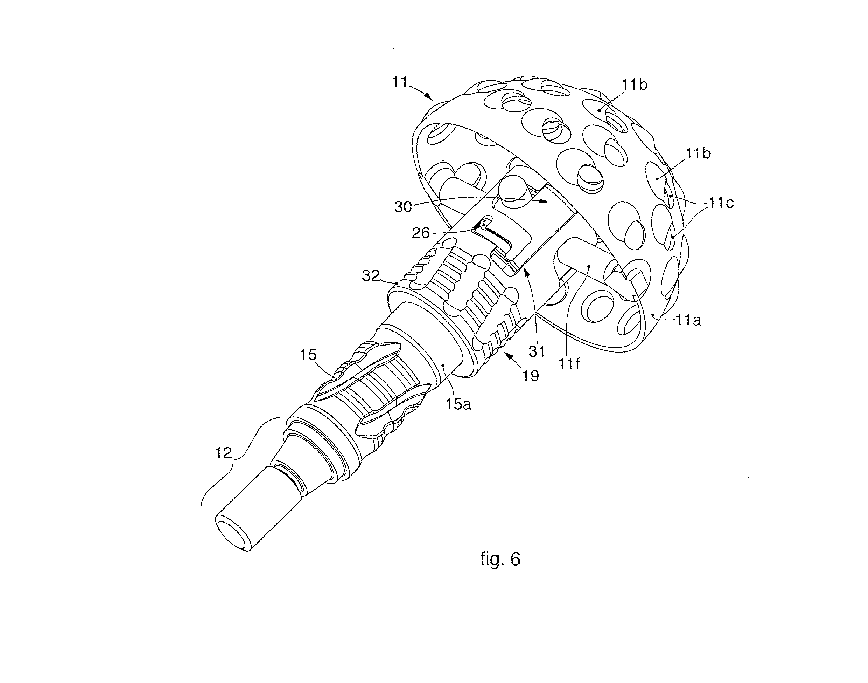

In other forms of embodiment the attachment unit 11d can have a cross-shaped conformation for example, in which the cap 11f has four radial arms, disposed at an angle of 90° with respect to each other and extending from the center toward the periphery, joining with the milling cap 11a (see fig. 6 for example).

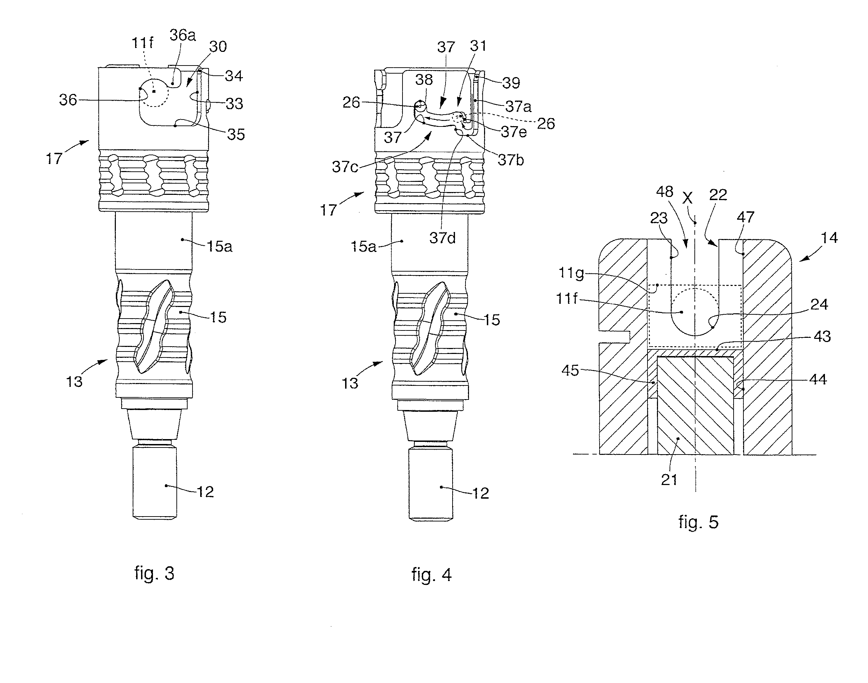

A central body 11 g may also be provided, in the medial part of the attachment unit 11d, and is shown schematically for convenience only in fig. 5. The central body 11g can also have a lower contact surface, to cooperate with the connection device 10, and can be made for example of ferromagnetic material.

According to the present description and with reference to the forms of embodiment described for example using figs. 1 - 6, the positioning unit 13 can be made as a single body and, in particular, at the opposite end to the end where the connection element 12 to the manipulator device is provided, it can have the positioning block 14.

In possible implementations, the positioning block 14 can have a cylindrical shape, which extends along the milling axis X, or in general it can be axial-symmetrical around the milling axis X and can be hollow inside, defining an internal positioning cavity 48 for the attachment unit 11d.

In possible implementations, the positioning block 14 can have an external diameter such as to be compatible with the axial and angular sliding of the mobile anchoring body 19, for example in relation to the passage between the distal non-operative position, the proximal insertion position and the operative angular and axial clamping position.

In particular, the external diameter of the positioning block 14 can be such as to prevent the generation of friction between the positioning block 14 and the mobile anchoring body 19.

In possible forms of embodiment, the internal positioning cavity 48 defines a housing seating 47 configured to receive the attachment unit 11d.

In possible implementations, the housing seating 47 can include guide grooves or hollows 22, through which the radial arms of the cap 11 f can be inserted, and can also have a bottom 43 on which the attachment unit 11d of the milling tool 11 rests.

According to possible forms of embodiment described here, the bottom 43 of the housing seating 47 is such as to receive the attachment unit 11d, and in particular the central body 11g of the milling tool 11 at the moment when the latter is positioned on the positioning unit 13.

According to possible forms of embodiment, the positioning block 14 can include magnetic holding means 20, to hold the attachment unit 11d of the milling tool 11.

In particular, according to possible implementations, on the bottom 43 of the housing seating 47 a seating 44, for example blind, may be made to house magnets, able to receive and position one or more magnetic elements 21 of the magnetic holding means 20, to generate an axial traction magnetic force on the milling tool 11 toward the inside of the housing seating 47. Examples of magnetic elements are natural magnets.

In this way, by means of the magnetic holding means 20, it is possible to constrain the milling tool 11 to the connection device 10, mating this effect with that of the axial holding seatings 30 and the angular holding seatings 31 for clamping the milling tool 11.

According to some forms of embodiment, which can be combined with all the forms of embodiment described here, inside the bottom 43 a protective cover 45 may also be provided, disposed to cover the one or more magnetic elements 21, to protect it from knocks, and also to prevent it from being in contact with the external environment or to prevent particles thereof from being released to the outside, which may not be desired, for example in washing machines where such tools are normally processed.

In possible implementations, the protective cover 45 can be shaped and sized to couple with the magnet housing seating 44, separating the one or more magnetic elements 21 from the outside.

In possible implementations, the protective cover 45 can be inserted into the magnet housing seating 44 and positioned or fitted on the one or more magnetic elements 21, so as to contact the latter at the upper part.

The protective cover 45 on one side can determine, with its thickness, a positioning compensation for the one or more magnetic elements 21 and on the other side can define the bottom 43.

The bottom 43 can receive the attachment unit 11d of the milling tool 11 in abutment or support, in particular the base of the central body 11g.

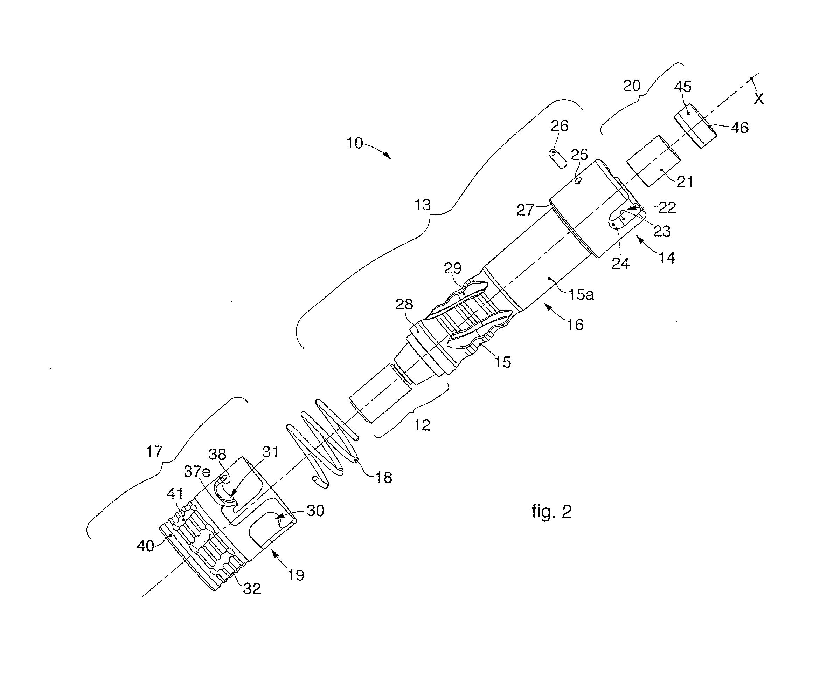

According to some forms of embodiment, the guide grooves 22, described with reference to fig. 2 for example, can have a development parallel to the milling axis X. In particular, the guide grooves 22 can be apertures having an upward concavity, more precisely facing toward the milling tool 11, in particular toward the radial arms of the cap 11f.

For example, the guide grooves 22 can be apertures made in the thickness of the walls of the positioning block 14, in particular made radially through.

According to some forms of embodiment, the guide grooves 22 can have a shape such as to receive the radial arms of the cap 11f of the milling tool 11 and subsequently to draw the attachment unit 11d into rotation and prevent a relative rotation of the milling tool 11 and the positioning unit 13 around the milling axis X.

A possible shape for this purpose can be, for example, a U shape or similar, with its opening facing toward the entrance of the radial arms of the cap 11f (see fig. 2). In general, symmetrical shapes can be provided, with nominal sizes essentially the same as those of the radial arms of the cap 11f, so as to contain the latter without any possibility of extra travel. Therefore, the guide grooves 22 can have other shapes, configured to prevent a relative rotation of the milling tool 11 and the positioning unit 13, without departing from the field of the present invention.

According to some forms of embodiment, the guide grooves 22 can be provided in a number mating with the number of the radial arms of the cap 11f, in this case, with reference for example to figs. 1 - 5, two guide grooves 22.

In other forms of embodiment, the positioning block 14 can include a larger number of guide grooves 22, in coordination with another conformation and number of the radial arms of the cap 11f of the milling tool 11, for example cross-shaped (see fig. 6 for example).

In yet other possible implementations, there can be three guide grooves 22, for example with a triangular shaped geometrical configuration. According to possible forms of embodiment, each of the guide grooves 22 can include guide walls 23, having a development parallel to the milling axis X which allow to insert the milling tool 11, and in particular the radial arms of the cap 11f, until a lower positioning seating 24 is reached, which corresponds to the point of maximum insertion of the radial arms of the cap 11f into the guide grooves 22.

In possible implementations, the lower positioning seating 24 can have a rounded shape, advantageously symmetrical and with a nominal size, in particular in diameter, essentially the same as that of the radial arms of the cap 11f. In other forms of embodiment, the positioning seating 24 can be flat and linear.

The correct positioning of the milling tool 11 in the guide grooves 22 can therefore prevent a relative rotation of the milling tool 11 and the positioning unit 13 around the milling axis X. The axial sliding of the milling tool 11, which would otherwise be possible, is instead prevented by the mobile anchoring body 19.

According to some forms of embodiment, the positioning block 14 can be provided with a clamping mean 26, such as for example a pin or a peg or similar clamping or stopping mean, advantageously configured to cooperate with the angular holding seating 31 and therefore allow to obtain the angular constraint of the positioning block 14 and the mobile anchoring body 19 (see for example fig. 2).

According to some forms of embodiment, the positioning block 14 can be provided with a housing 25, such as for example a fissure or hole, transverse to the milling axis X, made inside the thickness of the wall of the positioning block 14 and communicating with the outside through an aperture provided on the external surface of the positioning block 14. The housing 25 can be of a mating shape to receive the clamping mean 26.

According to possible forms of embodiment, as we said above, the positioning unit 13 can include the intermediate sliding guide body 16 and this can be connected in continuity with the positioning block 14 through a support surface 27, in particular an abutment or axial stop step.

In possible implementations, the support surface 27 can have an annular shape, coaxial to the milling axis X and can be configured so that its external diameter corresponds to the external diameter of the positioning block 14, whereas its internal diameter corresponds at least to the external diameter of the intermediate sliding guide body 16.

The support surface 27, as described hereafter as well, can have the purpose of blocking the axial advance of the elastic element 18, acting as an "end-of-travel".

According to possible forms of embodiment, the intermediate sliding guide body 16 can have an oblong shape, for example cylindrical.

In other forms of embodiment, the intermediate sliding guide body 16 can have different shapes, compatible with its main guide and sliding function, without departing from the field and scope of the present invention.

In some forms of embodiment, the intermediate sliding guide body 16 can include a handle 15, for a firm grip, and a sliding cylinder 15a, configured for suitable cooperation with the anchoring unit 17. The sliding cylinder 15a has a cylindrical shape that develops along the milling axis X. The external surface of the cylinder 15a can be subjected to various surface workings, preferably to define a surface with low roughness, essentially smooth and continuous, that facilitates the sliding of the elements of the anchoring unit 17.

In some forms of embodiment, the support surface 27 and the sliding cylinder 15a can have different types of surface working, without departing from the field of the present invention.

In possible implementations, the handle 15 can include a connector 28 with a preferably cylindrical development along the milling axis X, so as to be uniform with the remaining components of the positioning unit 13. The connector 28 can also have a suitable ergonomic shaping to adapt to the shape of the hand during the gripping action. In order to facilitate an easy and safe grip, the connector 28 can include anti-slip elements 29, such as for example elements of rubber or high-friction polymer material.

The positioning unit 13, as described above according to possible forms of embodiment, can be a single body, except for components that are expressly and totally removable, like the clamping mean 26; however, it does not have partly releasable components that would cause the formation of deposits in the dead angles of the connection device. This makes the cleaning and sanitization operations easier and safer.

According to some forms of embodiment, the mobile anchoring body 19 can have a mainly cylindrical shape, with an extension along the milling axis X, and can be hollow inside to achieve an axial and angular sliding with respect to the positioning unit 13. To this purpose, the internal diameter of the mobile anchoring body 19 can be at least equal to the external diameter of the positioning block 14, which represents the maximum diameter of the positioning unit 13. The mobile anchoring body 19 includes, as we said, the axial holding seatings 30, the angular holding seating 31 and a handle portion 32. The handle portion 32 can include a gripping body 40 with a cylindrical shape compatible with the mobile anchoring body 19 and anti-slip segments 41 to guarantee the correct grip of the mobile anchoring body 19 by the user.

According to some forms of embodiment, the axial holding seatings 30, for example two, three, four or more, can be made in the thickness of the walls of the mobile anchoring body 19.

In some forms of embodiment, the axial holding seatings 30 can be equal in number to the radial arms of the cap 11f and the guide grooves 22. In this case, with reference to figs. 1-5, the number of axial holding seatings 30 can be two. In other forms of embodiment, the number of axial holding seatings 30 can be higher, for example four (see fig. 6).

In possible implementations, the axial holding seatings 30 can be through apertures made radially, facing toward the radial arms of the cap 11 f in an axial direction, with an open peripheral profile. The axial holding seatings 30 have an amplitude such as to allow the insertion and sliding of the radial arms of the cap 11f, until they reach a housing portion 36 associated with an operative angular and axial clamping position (see fig. 3 for example). The housing portion 36 can have a widened end 36a, to angularly clamp the radial arm of the housed cap 11f.

According to some forms of embodiment, the axial holding seatings 30 also include an insertion segment 33 with a development parallel to the milling axis X and a transverse segment 35 that joins the insertion segment 33 to the housing portion 36 (see fig. 3 for example), defining an insertion path that the radial arm of the cap 11f must follow, for example substantially U-shaped.

In some forms of embodiment, the insertion segment 33 can provide a lead-in 34, for example a flare, in a direction parallel to the milling axis X, advantageously beveled, to facilitate the entrance of the radial arm of the cap 11f inside the axial holding seating 30.

According to some forms of embodiment, the insertion segment 33, the transverse segment 35 and the housing portion 36 can be connected to each other with a connection radius such as to assist the movement of the radial arms of the cap 11f, facilitating insertion and preventing it from jamming inside the axial holding seating 30.

Starting from said distal non-operative position, it is possible to take the mobile anchoring body 19 to the proximal insertion position, maintained by the cooperation between the clamping mean 26 and the angular holding seating 31, for example by mechanical interference. In this position, the guide grooves 22 are aligned in a radial direction with the insertion segments 33 and allow to insert the radial arms of the cap 11f. It is then possible, by rotating the mobile anchoring body 19 around the milling axis X, to take the housing portion 36 to abut on the radial arms of the cap 11f. In this condition, the radial arms of the cap 11f obtain the operative angular and axial clamping position.

In order to prevent the accidental dis-insertion of the radial arms of the cap 11f from the housing portion 36, in the event of sudden or involuntary maneuvers or simple thrust and rotation actions, the mobile anchoring body 19 includes the angular holding seating 31. As we said, the angular holding seating 31 prevents any unwanted rotation of the mobile anchoring body 19 and thus prevents it from returning to the proximal insertion position, in which it would be free to detach itself axially from the attachment unit 11d.

In particular, the angular holding seating 31 can be an aperture with an open peripheral profile, made in the thickness of the walls of the mobile anchoring body 19, radially through and with an entrance 39 facing toward the milling tool 11 in an axial direction with respect to the milling axis X (see fig. 4 for example).

According to some forms of embodiment, the angular holding seating 31 includes an attachment path 37, which at the end has a safety housing 38. At the beginning the attachment path 37 has said entrance 39, facing in an axial direction toward the milling tool 11. The entrance 39 can be provided with a lead-in to facilitate the entry of the clamping mean 26 inside the attachment path 37.

The attachment path 37 and the safety housing 38 can have an amplitude such as to stably guide the insertion and sliding of the clamping mean 26, essentially without resistance, except that due to the changes in direction imposed by the attachment path 37.

According to some forms of embodiment, the attachment path 37 includes an axial segment 37a with a length coordinated to that of the insertion segment 33 of the axial holding seatings 30, a transverse segment 37b of suitable length, and correlated to the amplitude of the rotation needed for coupling, and a connection segment 37c that joins the transverse segment 37b and the safety housing 38.

In possible implementations, the connection segment 37c, which can be for example substantially S-shaped, can have at the beginning a widened end 37d and a stepped seating 37e, respectively to make the entrance of the clamping mean 26 univocal into the connection segment 37c and to define the proximal insertion position. Moreover, the connection segment 37c can have a connection segment 37f between the stepped seating 37e and the safety housing 38.

In particular, the stepped seating 37e can be configured so as to house the clamping mean 26, to allow the axial insertion of the milling tool 11.

In particular, the clamping mean 26 reaches the proximal insertion position, as shown by dashes in fig. 4, after having traveled the axial segment 37a and the transverse segment 37b and after abutting against the stepped seating 37e, as shown schematically by way of example by the arrows in fig. 4. It is only in the proximal insertion position therefore that the axial holding seatings 30 and in particular the corresponding insertion segments 33 are aligned with the guide grooves 22, thus allowing the axial passage and insertion of the radial arms of the cap 11f. This can allow to replace the milling tool 11, for example, during a surgical operation without needing to assume the distal non-operative position, which is typically assumed for example for washing operations.

According to some forms of embodiment described here, in the proximal insertion position the mobile anchoring body 19 can rotate only in a clockwise direction, due to the specific configuration of the stepped seating 37e, which blocks its rotation in the anti-clockwise direction. Furthermore, the action of the elastic element 18 also contributes to this blocking action, which advantageously keeps the stepped seating 37e in abutment against the clamping mean 26.

In some forms of embodiment, the length of the insertion segment 33 of the axial holding seating 30 can be equal to the length of the axial segment 37a of the angular holding seating 31, or it can be different, also depending on the relative sizes of the clamping mean 26 and the radial arm of the cap 11f.

According to some forms of embodiment, the attachment path 37 is configured to receive and allow the insertion of the clamping mean 26; furthermore, its amplitude is such as to allow the clamping mean 26 to travel along the attachment path 37 until it reaches the operative angular and axial clamping position associated with the positioning of the clamping mean 26 in the safety housing 38.

In particular, the clamping mean 26 reaches the operative angular and axial clamping position, as shown by a continuous line in fig. 4, after having traveled along the connection segment 37c and abutting against the safety housing 38, as shown schematically by way of example, in this case too, by the arrows in fig. 4. It is only in the operative angular and axial clamping position, therefore, that the angular holding seatings 31, and in particular the corresponding widened ends 36a in the housing portion 36, receive and clamp the radial arms of the cap 11f. In this case too, the action of the elastic element 18 cooperates to keep the housing portion 36 thrust in abutment against the relative radial arm of the cap 11f.

The clamping mean 26 disposed in the safety housing 38 allows to prevent possible accidental dis-insertions of the radial arms of the cap 11f from the axial holding seatings 30, and therefore at the same time to prevent axial sliding and rotation around the milling axis X.

In the passage between the proximal insertion position and the operative angular and axial clamping position, since the radial arms of the cap 11f can have a bigger diameter than the clamping mean 26 and hence also a greater bulk and difficulty in passing, the axial holding seating 30 does not exactly reproduce the attachment path 37 but on the contrary allows a desired play of the radial arm of the cap 11f when the clamping mean 26 is moving along the attachment path 37. However, the axial holding seating 30 guarantees that the operative angular and axial clamping position is reached simultaneously both for the clamping mean 26 and also for the radial arms of the cap 11f, and that both these elements are stably inserted respectively into the safety housing 38 and the housing portion 36.

To this purpose, the length of the transverse segment 35 and the connection segment 37c is advantageously the same size. With reference to possible implementations concerning the elastic element 18, it can be completely separable from the mobile anchoring body 19, and therefore easily washable. Furthermore, in the operative angular and axial clamping position, the elastic element 18 can abut against the support surface 27 and can be contained at another end by the mobile anchoring body 19, and in particular by a corresponding lower abutment wall 42. Inside the portion of space available between the support surface 27 and the lower abutment wall 42, the elastic element 18 is normally pre-compressed, generating an axial thrust in the direction of the lower abutment wall 42 which in practice thrusts the whole mobile anchoring body 19 in this direction, allowing the clamping mean 26 and the radial arms of the cap 11 f to remain, respectively, abutting in the safety housing 38 and the axial holding seating 30.

In possible implementations, the lower abutment wall 42 (visible for example in fig. 1) can have an annular shape, with an external diameter equal to or greater than that of the mobile anchoring body 19, and an internal diameter at least equal to that of the intermediate sliding guide body 16 of the positioning unit 13.

According to some forms of embodiment, the presence of the elastic element 18 can be advantageous, since it allows not only to reach the operative angular and axial clamping position, but also to maintain it.

In possible forms of embodiment, described for example using fig. 6, the milling tool 11 can have a cross-shaped attachment unit 11d, provided with four radial arms of the cap 11f. In this variant, the positioning block 14 and the mobile anchoring body 19 are also made in coordination with the cross-shaped geometry. In particular, to this purpose the positioning block 14 includes four guide grooves 22 and in the same way the mobile anchoring body 19 can also be configured with a number of axial holding seatings 30 such as to allow to house all the radial arms of the cap 11 f. It is clear that, in the variant with the cross-shaped geometry, since a higher number of axial holding seatings 30 can be provided, the attachment path 37 that cooperates with the clamping mean 26 can be varied, according to needs, with respect to the configuration with two radial arms of the cap 11f, while still remaining within the field of the present invention.

It is clear that modifications and/or additions of parts may be made to the connection device 10 as described heretofore, without departing from the field and scope of the present invention.

It is also clear that, although the present invention has been described with reference to some specific examples, a person of skill in the art shall certainly be able to achieve many other equivalent forms of connection device, having the characteristics as set forth in the claims and hence all coming within the field of protection defined thereby.

## Claims

1. Connection device to releasably connect a milling tool (11) for prosthetic surgery and a manipulator device, **characterized in that** it comprises:
a positioning unit (13) configured to receive an attachment unit (11d) of the milling tool (11) and to be connected to the manipulator device to be made to rotate around a milling axis (X),
an anchoring unit (17), configured to anchor axially and angularly releasably the milling tool (11) to the positioning unit (13), wherein said anchoring unit (17) comprises a mobile anchoring body (19) provided with at least one axial holding seating (30) configured to receive and axially clamp the attachment unit (11d) of the milling tool (11), and with at least one angular holding seating (31) configured to cooperate with the positioning unit (13) in an operative angular and axial clamping position, wherein a complete releasable clamping condition is defined of the attachment unit (11d) to the positioning unit (13), so as to prevent an unwanted rotation of the mobile anchoring body (19).

2. Connection device as in claim 1, **characterized in that** the anchoring unit (17) is configured mobile with respect to the positioning unit (13), axially along said milling axis (X) and angularly around it, to selectively define the condition of complete releasable clamping of the attachment unit (11d) to the positioning unit (13) and a condition of complete detachment.

3. Connection device as in claim 2, **characterized in that** the anchoring unit (17) is mobile axially and angularly along the positioning unit (13) between a distal non-operative position, in which it can be completely detached and separated, and a proximal insertion condition, in which it can axially receive the attachment unit (11d) of the milling tool (11), and is mobile angularly around the positioning unit (13), between the proximal insertion position and the operative angular and axial clamping position, which defines said condition of complete releasable clamping of the attachment unit (11d) to the positioning unit (13).

4. Connection device as in any claim hereinbefore, **characterized in that** the anchoring unit (17) comprises an elastic element (18) configured to act elastically along the milling axis (X) and abut on one side against the mobile anchoring body (19) and on the other against the positioning unit (13).

5. Connection device as in claim 4, **characterized in that** the elastic element (18) is configured to thrust normally against the mobile anchoring body (19) along the milling axis (X).

6. Connection device as in any claim hereinbefore, **characterized in that** the positioning unit (13) comprises a positioning block (14) configured to receive the attachment unit (11d) of the milling tool (11).

7. Connection device as in claim 6, **characterized in that** the positioning block (14) is configured to draw into rotation the attachment unit (11d) and prevent any relative rotation between the milling tool (11) and the positioning unit (13) around the milling axis (X).

8. Connection device as in claim 6 or 7, **characterized in that** , the positioning block (14) is provided with a clamping mean (26) configured to cooperate with said angular holding seating (31).

9. Connection device as in claim 8, **characterized in that** the at least one angular holding seating (31) comprises a stepped seating (37e), configured to house said clamping mean (26), determining the proximal insertion position of the anchoring unit (17), and a safety housing (38), configured to house said clamping mean (26), determining the operative angular and axial clamping position of the anchoring unit (17).

10. Connection device as in any claim hereinbefore, **characterized in that** the positioning unit (13) comprises an intermediate sliding guide body (16) to cooperate with the anchoring unit (17) and allow both sliding of the anchoring unit (17) with respect to the positioning unit (13) along the milling axis (X), and also a rotation of the anchoring unit (17) with respect to the positioning unit (13) around the milling axis (X).

11. Surgical instrument for prosthetic surgery comprising a milling tool (11) for prosthetic surgery, a manipulator device configured to make the milling tool rotate around a milling axis (X) and a connection device (10) according to any claim hereinbefore, to releasably connect the milling tool (11) and the manipulator device.
